# EUROPÄISCHE PATENTSCHRIFT

(11) **EP 3 856 059 B1**
(45) Veröffentlichungstag und Bekanntmachung des Hinweises auf die Patenterteilung: **22.01.2025**
(21) Anmeldenummer: 19779354.0
(22) Anmeldetag: 24.09.2019
(51) Int. Cl.: A61B 17/80

(54) **FIXATIONSSYSTEM FÜR KNOCHEN**
FIXATION SYSTEM FOR BONES
SYSTÈME DE FIXATION D'OS

(30) Priorität: 24.09.2018 AT 508152018
(43) Veröffentlichungstag der Anmeldung: 04.08.2021
(73) Patentinhaber: Hofall Holding 1 GmbH, 1010 Wien (AT)
(72) Erfinder: MAIER, Karin, 8280 Fürstenfeld (AT); MAIER, Christian, 8280 Fürstenfeld (AT); CLEMENT, Hans Gunther, 8047 Graz (AT)
(74) Vertreter: SONN Patentanwälte GmbH & Co KG
(86) Internationale Anmeldenummer: PCT/AT2019/060312
(87) Internationale Veröffentlichungsnummer: WO 2020/061601

(56) Entgegenhaltungen:
- EP-A1- 2 364 658
- WO-A1-2013/053539
- DE-A1- 102009 016 394

## Beschreibung

Die Erfindung betrifft ein Fixationssystem für einen Knochen gemäß dem Oberbegriff von Anspruch 1.

In der Plattenosteosynthese werden Knochenplatten und Knochenschrauben zur operativen Versorgung von Knochenverletzungen, insbesondere komplizierten Knochenfrakturen, eingesetzt. Besondere Bedeutung kommt dabei der Frakturkompression zu: Der Einsatz spezieller Knochenplatten ermöglicht nicht nur die Fixierung der Knochenfragmente, sondern auch das Verschieben der Knochenfragmente relativ zueinander.

Knochenplatten, die eine Frakturkompression ermöglichen, sind allgemein bekannt und werden routinemäßig eingesetzt. Beispielsweise können geeignete Knochenplatten ovale Durchgangslöcher aufweisen in die Knochenschrauben exzentrisch eingesetzt werden können. Beim Eindrehen zentriert sich die Knochenschraube sukzessive im Durchgangsloch. Da die Schraube bereits mit dem Knochen verbunden ist, verschiebt sich dabei der Knochen relativ zur Knochenplatte. Zwei Knochenfragmente können so zueinander verschoben werden und eine Fraktur komprimiert werden.

Aus dem Stand der Technik sind weiters Fixationssysteme für Knochen bekannt, die neben einer Frakturkompression eine winkelstabile Verbindung zwischen Knochenschraube und Knochenplatte ermöglichen. Eine winkelstabile Verbindung bezeichnet üblicherweise eine kraftschlüssige oder stoffschlüssige Verbindung zwischen Knochenschraube und Knochenplatte und wird auch als "Verblockung" bezeichnet.

Die DE 10 2010 025 001 A1 und die WO 2011/160846 A1 beschreiben Knochenplatten, die Durchgangslöcher mit einer umlaufenden radialen Rippe aufweisen. Diese Rippe ermöglicht eine Verklemmung mit dem Kopfgewinde einer Knochenschraube und damit eine winkelstabile Verbindung. Zusätzlich weisen die beschriebenen Durchgangslöcher eine kegelstumpfförmige Ausbildung auf, die sich zur Plattenunterseite hin verjüngt und die bei Verwendung einer Knochenschraube mit konischer Sitzfläche eine Verschiebung der Knochenplatte relativ zum Knochen ermöglicht. Die beschriebenen Knochenplatten ermöglichen bei der Verwendung mit Knochenschrauben mit Kopfgewinde und konischer Sitzfläche also eine Frakturkompression und Winkelstabilität, die Knochenschraube muss hierfür aber parallel zur Lochachse angesetzt werden.

Aus dem Stand der Technik sind auch Fixationssysteme für Knochen bekannt, die eine Frakturkompression ermöglichen und bei denen die Knochenschraube in einem Schrägwinkel zur Lochachse mit der Knochenplatte verbunden werden kann. In diesem Fall spricht man auch von einer polyaxialen oder multidirektionalen Verbindung zwischen Knochenschraube und Knochenplatte.

Die EP 2 364 658 A1 offenbart z.B. Knochenplatten mit Durchgangslöchern, die gleichzeitig eine Relativverschiebung von Knochenplatte und Knochen und eine polyaxiale Verbindung zwischen Knochenschraube und Knochenplatte ermöglichen. Zu diesem Zweck müssen bei den beschriebenen Knochenplatten zwingend Knochenschrauben mit sphärischer Sitzfläche ohne Kopfgewinde eingesetzt werden. Die beschriebenen Knochenplatten können außerdem mit Schrauben mit konischer Sitzfläche und Kopfgewinde verwendet werden. In diesem Fall ist eine winkelstabile Verbindung möglich, jedoch keine Polyaxialität.

Aus dem Stand der Technik bekannt sind außerdem Fixationssysteme für Knochen, die gleichzeitig winkelstabile und polyaxiale Verbindungen von Knochenschraube und Knochenplatte ermöglichen. Beispielsweise beschreibt die DE 43 43 117 A1 ein derartiges Fixationssystem. Die Winkelstabilität wird dadurch erreicht, dass beim Eindrehen der Knochenschraube eine Materialumformung stattfindet und eine Gewindeverbindung zwischen einem vorgeformten Gewinde an Knochenschraube oder Knochenplatte mit der jeweils anderen Komponente gebildet wird. Allerdings kann das beschriebene Fixationssystem nicht zur Frakturkompression eingesetzt werden.

Vorteilhaft wäre ein Fixationssystem für Knochen, das eine Frakturkompression ermöglicht und gleichzeitig eine winkelstabil-polyaxiale Verbindung zwischen Knochenschraube und Knochenplatte erlaubt. Einige aus dem Stand der Technik bekannte Fixationssysteme versuchen diese Aufgabe zu lösen, allerdings weisen diese bekannten Fixationssysteme erhebliche Nachteile auf.

Die WO 00/66012 A1 versucht diese Aufgabe durch die Verwendung von speziellen Knochenschrauben zu lösen, die zwischen einem Schraubenkopf mit glatter Sitzfläche und dem gewöhnlichen Schraubengewinde am Schraubenschaft ein kurzes zusätzliches Verblockungsgewinde am Schraubenhals aufweisen. Das Verblockungsgewinde kann sich mit einer Eingriffkontur in der Knochenplatte verschrauben und die Knochenschraube winkelstabil fixieren. Außerdem soll die interne Eingriffskontur einen leichten Winkelversatz der eingedrehten Schraube zur Lochachse erlauben. Um eine Frakturkompression zu erreichen, soll die Schraube exzentrisch in ein Langloch eingebracht werden. Ein erheblicher Nachteil der beschriebenen Knochenplatten und Knochenschrauben ist allerdings, dass Polyaxialität nur in einem sehr begrenzten Ausmaß gegeben ist, d.h. dass nur ein leichter Winkelversatz der eingebrachten Knochenschraube möglich ist.

Die DE 198 58 889 A1 versucht die Aufgabe durch den Einsatz ähnlicher Knochenschrauben zu lösen. Die beschriebenen Knochenschrauben haben ebenfalls einen Schraubenkopf mit glatter Sitzfläche sowie ein zusätzliches Verblockungsgewinde zwischen Schraubenkopf und Schraubenschaft. Eine der beschriebenen Ausführungsformen der Knochenplatten weist längliche Durchgangslöcher auf, die eine Frakturkompression ermöglichen sollen. Dabei soll es möglich sein, die Knochenschraube in einem Schrägwinkel einzubringen. Außerdem soll ein umformbarer Vorsprung im Durchgangsloch in Zusammenwirkung mit dem Verblockungsgewinde der Knochenschraube eine winkelstabile Verbindung ermöglichen. Ein erheblicher Nachteil des beschriebenen Fixationssystem ist allerdings, dass die Winkelstabilität nur in sehr niedrigem Ausmaß gegeben sein kann. Die Knochenschraube ist im eingedrehten Zustand über höchstens 180° ihres Umfangs mit der Wand des länglichen Durchgangsloches in Kontakt. Da sie sich nicht über ihren gesamten Umfang mit dem umformbaren Vorsprung verschrauben kann, kann keine hohe Winkelstabilität erreicht werden.

Es besteht daher weiterhin ein Bedarf an einem Fixationssystem für Knochen, das eine Verschiebung von Knochen relativ zur Knochenplatte und gleichzeitig eine winkelstabil-polyaxiale Verbindung zwischen Knochenplatte und Knochenschraube ermöglicht.

Die Aufgabe der vorliegenden Erfindung besteht darin, zumindest einzelne Nachteile des Standes der Technik zu lindern bzw. zu beseitigen. Die Erfindung setzt sich insbesondere zum Ziel, ein Fixationssystem für Knochen bereitzustellen, das eine Verschiebung von Knochen relativ zur Knochenplatte und gleichzeitig eine winkelstabil-polyaxiale Verbindung zwischen Knochenplatte und Knochenschraube ermöglicht.

Diese Aufgabe wird durch ein Fixationssystem mit den Merkmalen von Anspruch 1 gelöst.

Erfindungsgemäß weist das Durchgangsloch einen Führungsabschnitt auf, der sich von der Schraubeneintrittsöffnung in Richtung der Schraubenaustrittsöffnung verjüngt, sodass die im Wesentlichen sphärische Sitzfläche des Schraubenkopfes beim Eindrehen der Knochenschraube in den Knochen, unter Verschiebung der Knochenplatte, entlang einer Führungsfläche des Führungsabschnitts gleitet und in den Verblockungsabschnitt geführt wird.

Die Erfindung ermöglicht somit gleichzeitig den Einsatz der Knochenschraube in unterschiedlichen Schrägwinkeln zur Lochachse (Polyaxialität), eine winkelstabile Verbindung zwischen Knochenplatte und Knochenschraube in diesen Schrägwinkeln und eine Verschiebung der Knochenplatte relativ zum Knochen beim Eindrehen der Knochenschraube.

Erfindungsgemäß ist vorgesehen, dass die sphärische Sitzfläche und das Kopfgewinde der Knochenschraube eine Einheit bilden. D.h. die Knochenschraube hat keine zusätzliche Sitzfläche oberhalb des Kopfgewindes. Anders ausgedrückt, ist das Kopfgewinde der Knochenschraube Teil der sphärischen Sitzfläche des Schraubenkopfes. Das hat den Vorteil, dass eine hohe Polyaxialität gegeben ist, so dass die Knochenschraube in einem großen Winkelbereich eingesetzt werden kann. Die Knochenschraube kann daher in unterschiedlichen Schrägwinkeln zur Lochachse winkelstabil im sich vorzugsweise an der Plattenunterseite befindenden Verblockungsabschnitt des Durchgangsloches fixiert werden.

Die Führungsfläche des erfindungsgemäßen Durchgangsloches ermöglicht eine Verschiebung der Knochenplatte relativ zum Knochen beim Eindrehen der Knochenschraube. Die Verschiebung findet dabei in eine Richtung quer zur Lochachse, d.h. in eine Richtung in der Plattenebene statt. So kann beispielsweise der Abstand zwischen zwei Knochenfragmenten verringert werden und eine Frakturkompression erreicht werden. Besonders vorteilhaft ist es, wenn die Führungsfläche im Wesentlichen glattwandig ist.

In einer bevorzugten Ausführungsform stellt der Verblockungsabschnitt im Querschnitt gesehen einen Kreis dar. In einer besonders bevorzugten Ausführungsform ist der Verblockungsabschnitt im Wesentlichen zylindrisch. Dadurch wird ermöglicht, dass der Schraubenkopf im verblockten Zustand im Querschnitt gesehen über den gesamten Umfang des Verblockungsabschnitts an den Wänden des Durchgangsloches anliegen kann. Anders ausgedrückt kann der Schraubenkopf in der Plattenebene gesehen von allen Seiten fixiert werden. Somit ist eine besonders stabile Verblockung der Knochenschraube möglich.

In einer weiteren bevorzugten Ausführungsform ist der Verblockungsabschnitt im Wesentlichen konisch. Der Konuswinkel kann dabei zwischen 1° und 25°, vorzugsweise zwischen 4° und 20°, noch mehr bevorzugt zwischen 8° und 20°, am meisten bevorzugt zwischen 12° und 16° betragen. Dadurch wird das Anbeißverhalten des Schraubenkopfes verbessert.

Vorzugsweise erstreckt sich der umformbare Vorsprung im Querschnitt gesehen über den gesamten Umfang des Verblockungsabschnitts. Das hat den Vorteil, dass sich der Schraubenkopf im Querschnitt gesehen über den gesamten Umfang des Verblocksabschnitts verschrauben kann, wodurch eine besonders stabile Verblockung erreicht wird.

Besonders bevorzugt ist es, wenn der umformbare Vorsprung Teil eines umformbaren Gewindes ist. Das Gewinde kann vorzugsweise mindestens eine volle Umdrehung, noch mehr bevorzugt mindestens zwei volle Umdrehungen aufweisen. Somit kann sich der Schraubenkopf besonders stabil im Verblockungsabschnitt verschrauben.

In einer bevorzugten Ausführungsform der vorliegenden Erfindung weist der Führungsabschnitt eine zur Lochachse parallele, d.h. senkrecht zur Plattenebene erstreckte, Eingangsfläche auf.

Bevorzugt ist es außerdem, wenn der Führungsabschnitt eine schräge, zum Verblockungsabschnitt führende Führungsfläche aufweist, welche vorzugsweise unmittelbar an die Eingangsfläche anschließt. Das ermöglicht ein einfaches Gleiten der Sitzfläche des Schraubenkopfes entlang der Führungsfläche beim Eindrehen der Knochenschraube. Die Führungsfläche ist vorzugsweise glattwandig. Damit wird ein besonders einfaches Gleiten der Sitzfläche des Schraubenkopfes ermöglicht. Die Führungsfläche kann alternativ auch gekrümmt sein. In einer bevorzugten Ausführungsform beträgt der Winkel der Führungsfläche zur Plattenebene im Längsschnitt (parallel zur Lochachse) gesehen zwischen 10° und 80°, vorzugsweise zwischen 20° und 70°, noch mehr bevorzugt zwischen 30° und 60°, noch mehr bevorzugt zwischen 40° und 50°.

Besonders bevorzugt ist es, wenn der Führungsabschnitt eine zur Lochachse parallele Eingangsfläche und eine schräge, zum Verblockungsabschnitt führende Führungsfläche aufweist.

In einer weiteren besonders bevorzugten Ausführungsform erstreckt sich die Führungsfläche bis zur Schraubenaustrittsöffnung an der Plattenoberseite. In dieser Ausführungsform weist der Führungsabschnitt keine abgesetzte Eingangsfläche auf.

In einer besonders bevorzugten Ausführungsform der vorliegenden Erfindung ist der Schraubenkopf im verblockten Zustand vollständig in dem Verblockungsabschnitt aufgenommen. Vollständig aufgenommen bedeutet in diesem Zusammenhang, dass im Längsschnitt gesehen, kein Teil des Schraubenkopfes über den Verblockungsabschnitt hinausragt. Die vollständige Aufnahme des Schraubenkopfes im Verblockungsabschnitt hat den Vorteil, dass der Schraubenkopf besonders stabil fixiert werden kann.

In einer besonders bevorzugten Ausführungsform ist als Schraubeneintrittsöffnung ein Langloch vorgesehen. Vorzugsweise entspricht die Breite des Langloches im Wesentlichen dem Durchmesser der Schraubenaustrittsöffnung. Die Breite bezeichnet in diesem Zusammenhang dem Abstand der parallel zueinander verlaufenden Längsseiten des Langloches. Vorzugsweise ist in dieser Ausführungsform die Ausdehnung des Durchgangsloches in Richtung der Breite des Langloches im gesamten Bereich zwischen Schraubeneintrittsöffnung und Schraubenaustrittsöffnung im Wesentlichen konstant. Das hat den Vorteil, dass die Verschiebung der Knochenplatte relativ zum Knochen beim Eindrehen der Knochenschraube nur in der Längsachse des Langloches möglich ist. Somit kann die Achse, in der die Verschiebung stattfinden soll, durch die Platzierung des Durchgangsloches in der Knochenplatte vorgegeben werden.

In einer weiteren bevorzugten Ausführungsform weist das Durchgangsloch eine sich zwischen der Schraubeneintrittsöffnung und der Schraubenaustrittsöffnung erstreckende zylindrisch geformte Seitenwand auf. Vorzugsweise ist in dieser Ausführungsform die Schraubeneintrittsöffnung ein Langloch und die Schraubenaustrittsöffnung ein Rundloch, wobei die Breite des Langloches dem Durchmesser des Rundloches entspricht. Vorzugsweise sind in dieser Ausführungsform die Schraubeneintrittsöffnung und die Schraubenaustrittsöffnung so übereinander positioniert, dass das die Schraubeneintrittsöffnung auf einer Seite abschließende Kreissegment im Längsschnitt gesehen im Wesentlichen parallel zu dem die Schraubenaustrittsöffnung beschreibenden Kreis angeordnet ist. Vorzugsweise ist der Querschnitt der zylindrischen Fläche ein Kreisbogen mit einem Mittelpunktswinkel von 180°. Diese Ausführungsform hat den Vorteil, dass die Verschiebung der Knochenplatte relativ zum Knochen beim Eindrehen der Knochenschraube nur in Richtung der Längsachse des Langloches möglich ist. Somit kann die Richtung, in der die Verschiebung stattfinden soll, durch die Platzierung des Durchgangsloches in der Knochenplatte vorgegeben werden. Ein weiterer Vorteil ist, dass eine sich zwischen der Schraubeneintrittsöffnung und der Schraubenaustrittsöffnung erstreckende zylindrisch geformte Seitenwand eine einfache Herstellung ermöglicht.

Vorzugsweise erstreckt sich der Verblockungsabschnitt entlang der Lochachse über 1/5 bis 4/5, noch mehr bevorzugt zwischen 2/5 und 4/5, noch mehr bevorzugt zwischen 3/5 und 4/5 der Distanz zwischen Schraubeneintrittsöffnung und der Schraubenaustrittsöffnung (d.h. der Längserstreckung des Durchgangsloches). Besonders bevorzugt ist es, wenn sich der Verblockungsabschnitt entlang der Lochachse über eine Distanz von mindestens 0,5 Millimeter, noch mehr bevorzugt mindestens 1 Millimeter, am meisten bevorzugt mindestens 1,5 Millimeter erstreckt.

Vorzugsweise erstreckt sich der Führungsabschnitt entlang der Lochachse über 1/5 bis 4/5, noch mehr bevorzugt zwischen 2/5 und 4/5, noch mehr bevorzugt zwischen 3/5 und 4/5 der Distanz zwischen Schraubeneintrittsöffnung und der Schraubenaustrittsöffnung. Besonders bevorzugt ist es, wenn sich der Führungsabschnitt entlang der Lochachse über eine Distanz von mindestens 0,25 Millimeter, noch mehr bevorzugt mindestens 0,5 Millimeter, am meisten bevorzugt mindestens 1,0 Millimeter erstreckt.

Die Knochenschrauben des erfindungsgemäßen Fixationssystems können z.B. Spongiosaschrauben oder Kortikalisschrauben sein. Spongiosaschrauben können in einem erfindungsgemäßen Fixationssystem in einem Winkelbereich bis zu 70° eingesetzt werden. Kortikalisschrauben können in einem Winkelbereich bis zu 45° eingesetzt werden.

Im Folgenden wird die Erfindung anhand von bevorzugten, nicht einschränkenden Ausführungsformen unter Bezugnahme auf die Zeichnungen näher erläutert. Es zeigen:
- Figur 1: ein erfindungsgemäßes Fixationssystem mit einer Knochenschraube und einer Knochenplatte vor der Verbindung miteinander;
- Figur 2A: die Knochenplatte aus Fig. 1 in Draufsicht;
- Fig. 2B: das Durchgangsloch der Knochenplatte in einer schematischen perspektivischen Darstellung;
- Figur 3A bis 3E: das Fixationssystem gemäß Fig. 1 in verschiedenen Stellungen beim Eindrehen der Knochenschraube senkrecht zur Plattenebene der Knochenplatte; und
- Figur 4A, 4B: jeweils das Fixationssystem gemäß Fig. 1 bis Fig. 3 in zwei Stellungen beim Eindrehen der Knochenschraube in einem Winkel zur Plattenebene der Knochenplatte.

Für die Zwecke dieser Offenbarung beziehen sich sämtliche Orts- und Richtungsangaben, wie "oben" und "unten" auf den bestimmungsgemäßen Gebrauchszustand, wenn die Knochenplatte auf dem Knochen aufliegt. Als "Plattenebene" ist die Hauptebene der Knochenplatte im Bereich des Durchgangsloches zu verstehen. Die folgenden Erläuterungen beziehen sich auf eine Ausführung, bei welcher die "Lochachse" senkrecht zur Plattenebene erstreckt ist. Die Lochachse kann jedoch auch schräg zur Plattenebene verlaufen, wobei die Richtungen dann entsprechend zu übertragen sind.

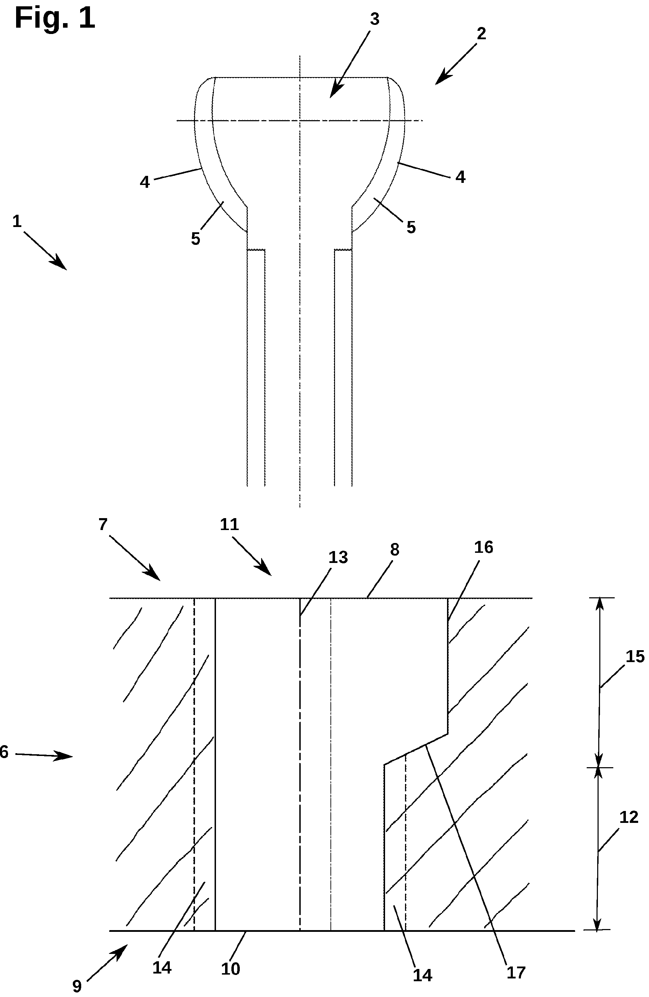

Fig. 1 zeigt ein Fixationssystem 1 für einen Knochen (nicht dargestellt). Das Fixationssystem 1 weist zum einen eine Knochenschraube 2 mit einem Schraubenkopf 3 mit einer im Wesentlichen sphärischen Sitzfläche 4 auf. An der Sitzfläche 4 ist zugleich ein Kopfgewinde 5 ausgebildet.

Das Fixationssystem 1 weist außerdem eine Knochenplatte 6 zur winkelstabil-polyaxialen Verblockung der Knochenschraube 2 auf. Die Knochenplatte 6 hat eine Plattenoberseite 7 mit einer Schraubeneintrittsöffnung 8 zum Einführen der Knochenschraube 2 und eine für den Knochenkontakt vorgesehene Plattenunterseite 9 mit einer Schraubenaustrittsöffnung 10. Zwischen der Schraubeneintrittsöffnung 8 und der Schraubenaustrittsöffnung 10 erstreckt sich ein Durchgangsloch 11.

Das Durchgangsloch 11 weist einen Verblockungsabschnitt 12 auf, der dazu dient, die Knochenschraube 2 in verschiedenen Winkeln zur Lochachse 13 stabil zu fixieren. Zu diesem Zweck weist der Verblockungsabschnitt 12 mindestens einen umformbaren Vorsprung 14 auf. Bei der in Fig. 1 dargestellten Ausführungsform ist der Verblockungsabschnitt 12 im Wesentlichen zylindrisch und weist ein umformbares Gewinde auf. Der umformbare Vorsprung 14 ist in dieser Ausfühungsform also Teil eines umformbaren Gewindes.

Das Durchgangloch 11 weist außerdem einen Führungsabschnitt 15 auf, der sich von der Schraubeneintrittsöffnung 8 in Richtung der Schraubenaustrittsöffnung 10 verjüngt. Bei der in Fig. 1 dargestellten Ausführungsform weist der Führungsabschnitt 15 eine zur Lochachse parallele Eingangsfläche 16 und eine schräge, zum Verblockungsabschnitt 12 führende Führungsfläche 17 auf.

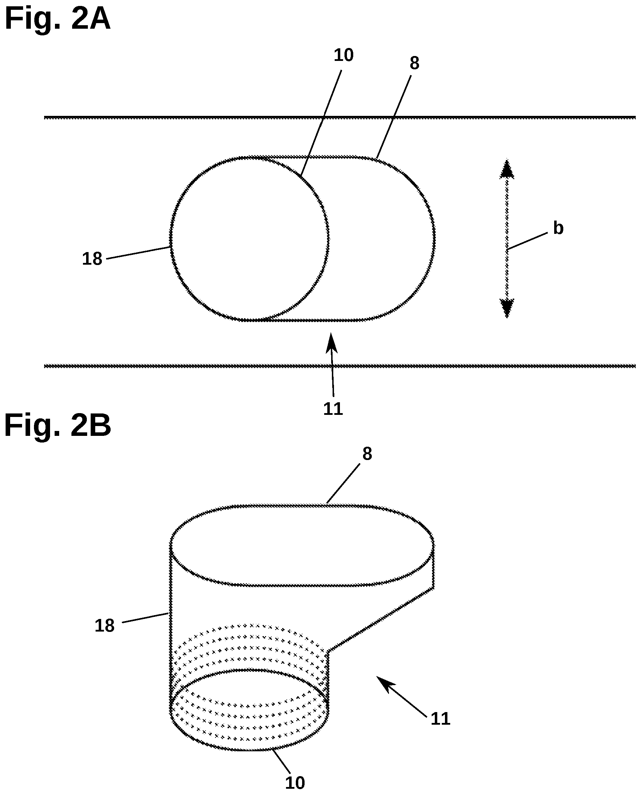

Fig. 2A zeigt schematisch eine Draufsicht und Fig. 2B zeigt eine schematische perspektivische Darstellung einer bevorzugten Ausführungsform des Durchgangslochs 11. In dieser Ausführungsform ist die Schraubeneintrittsöffnung 8 ein Langloch und die Schraubenaustrittsöffnung 10 ein Kreisloch. Die Breite b des Langloches entspricht dabei dem Durchmesser der Schraubenaustrittsöffnung 10. Die Ausdehnung des Durchgangsloches 11 in Richtung der Breite b des Langloches ist dabei im gesamten Bereich zwischen Schraubeneintrittsöffnung 8 und Schraubenaustrittsöffnung 10 konstant. In der in Fig. 2A, 2B dargestellten Ausführungsform weist das Durchgangsloch 11 außerdem eine sich zwischen der Schraubeneintrittsöffnung 8 und der Schraubenaustrittsöffnung 10 erstreckende zylindrisch geformte Seitenwand 18 auf, die im Querschnitt gesehen einen Kreisbogen mit einem Mittelpunktswinkel von 180° beschreibt.

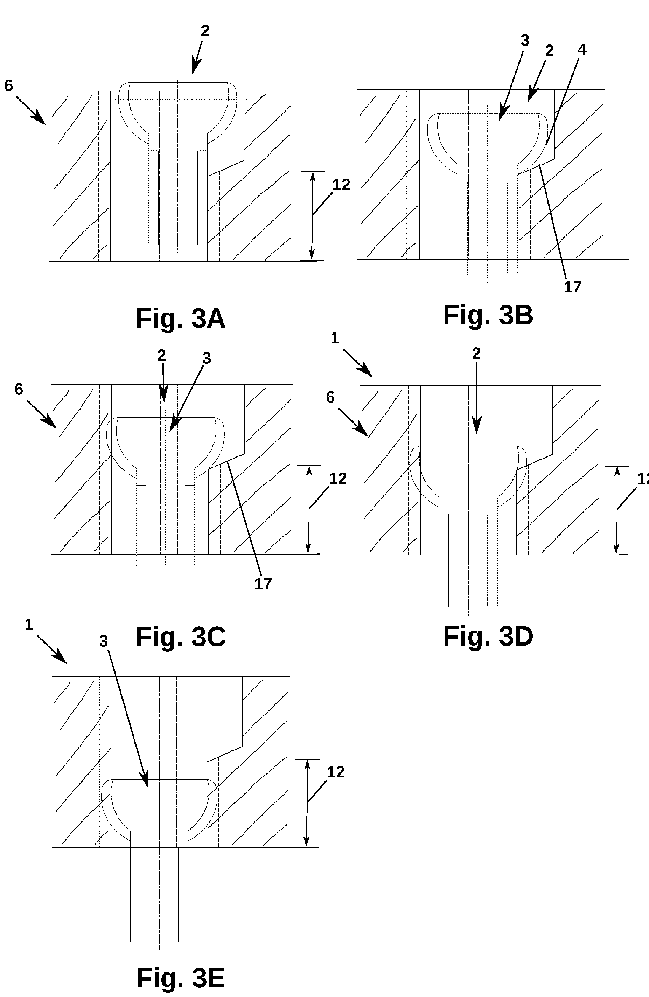

Fig. 3A bis 3E zeigt die Anwendung der in Figur 1 dargestellten Knochenschraube 2 und Knochenplatte 6. Fig. 3A bis 3E zeigen dabei aufeinanderfolgende Zustände beim Eindrehen der Knochenschraube 2.

Fig. 3A zeigt das Ansetzen der Knochenschraube 2. Um eine Verschiebung der Knochenplatte 6 relativ zum Knochen (nicht dargestellt) zu erreichen, wird die Knochenschraube 2 in Bezug auf den Verblockungsabschnitt 12 außermittig angesetzt. In Fig. 3B ist die Knochenschraube 2 bis zu dem Punkt eingedreht, an dem der Schraubenkopf 3 mit seiner Sitzfläche 4 an der Führungsfläche 17 ansteht. Fig. 3C zeigt wie sich die Knochenplatte 6 durch das Gleiten des Schraubenkopfes 3 entlang der Führungsfläche 17 verschiebt. Die Knochenschraube 2 bewegt sich beim Eindrehen sukzessive vom Rand des Verblockungsabschnitts 12 in dessen Mitte. Da die Knochenschraube 2 bereits in den Knochen (nicht dargestellt) eingetreten ist, hat das eine Verschiebung der Knochenplatte 6 relativ zum Knochen (nicht dargestellt) zur Folge. Fig. 3D zeigt einen Zustand des Fixationssystems 1, in dem die Verschiebung der Knochenplatte 6 abgeschlossen ist. Die Knochenschraube 2 ist im Verblockungsabschnitt 12 zentriert aber noch nicht verblockt. Fig 3E zeigt den verblockten Zustand des Fixationssystems 1. Der Schraubenkopf 3 ist vollständig in den Verblockungsabschnitt 12 aufgenommen und die Knochenschraube 2 ist stabil verblockt.

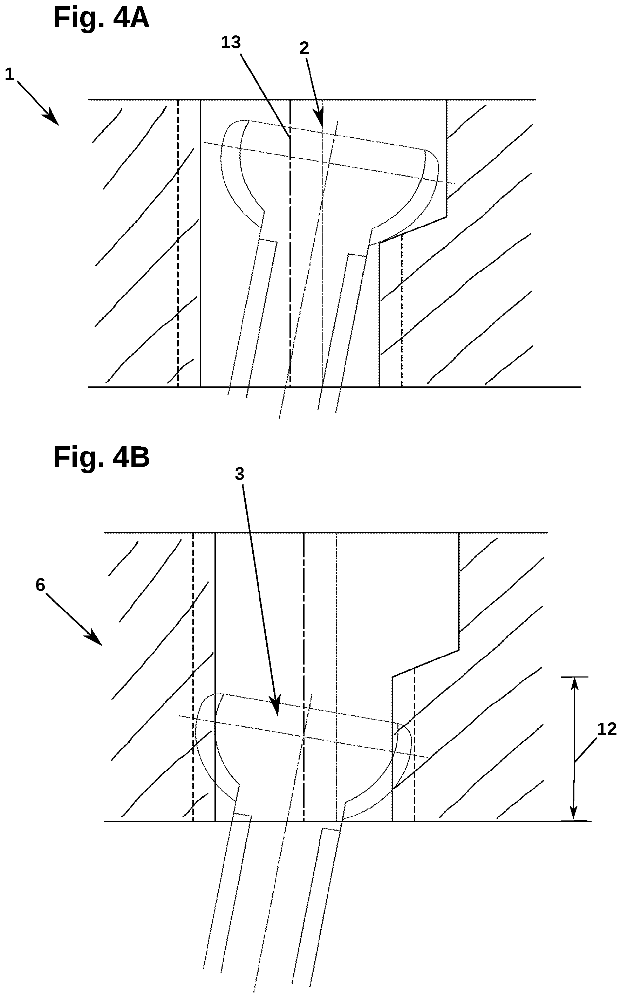

Fig. 4A zeigt das in Figur 1 dargestelle Fixationssystem 1, wobei die Knochenschraube 2 in einem Schrägwinkel zur Lochachse 13 eingebracht wurde. Fig. 4B zeigt den winkelstabil verblockten Zustand nachdem die Verschiebung der Knochenplatte 6 relativ zum Knochen (nicht dargestellt) erfolgt ist und der Schraubenkopf 3 in den Verblockungsabschnitt 12 eingetreten ist.

Das Fixationssystems kann wie folgt zur Kompression einer Knochenfraktur eingesetzt werden: Die Knochenplatte wird auf ein zu fixierendes Knochenstück aufgelegt und mit einer Knochenschraube mit dem Knochen verschraubt. Dabei kann die Knochenschraube entweder parallel zur Lochachse oder in einem Schrägwinkel eingesetzt werden. Nach dem vollständigen Eindrehen ist die Knochenschraube winkelstabil verblockt. Um eine Frakturkompression zu erreichen, wird die Knochenplatte mit einem weiteren Knochenstück verschraubt. Dabei wird die Knochenschraube auf den Verblockungsabschnitt bezogen exzentrisch angesetzt und entweder parallel zur Lochachse oder in einem Schrägwinkel in den Knochen eingedreht. Durch das sukzessive Zentrieren der Knochenschraube im Verblockungsabschnitt verschiebt sich das mit der Knochenschraube verbundene Knochenstück in Richtung des bereits an der Knochenplatte fixierten Knochenstücks. Nach dem vollständigen Eindrehen ist die Knochenschraube winkelstabil verblockt und die Frakturkompression ist vollzogen.

## Patentansprüche

1. Fixationssystem (1) für einen Knochen umfassend:
- eine Knochenschraube (2) mit einem Schraubenkopf (3) mit einer im Wesentlichen sphärischen Sitzfläche (4), an welcher ein Kopfgewinde (5) ausgebildet ist und
- eine Knochenplatte (6) zur winkelstabil-polyaxialen Verblockung der Knochenschraube (2), aufweisend:
eine Plattenoberseite (7) mit einer Schraubeneintrittsöffnung (8) zum Einführen der Knochenschraube (2),
eine für den Knochenkontakt vorgesehene Plattenunterseite (9) mit einer Schraubenaustrittsöffnung (10),
ein sich zwischen der Schraubeneintrittsöffnung (8) und der Schraubenaustrittsöffnung (10) erstreckendes Durchgangsloch (11),
einen Verblockungsabschnitt (12), der mindestens einen durch Eindrehen der Knochenschraube (2) umformbaren Vorsprung (14) zum stabilen Verblocken der Knochenschraube (2) in verschiedenen Winkeln zur Lochachse (13) aufweist,
**dadurch gekennzeichnet, dass**
das Durchgangsloch (11) einen Führungsabschnitt (15) aufweist, der sich von der Schraubeneintrittsöffnung (8) in Richtung der Schraubenaustrittsöffnung (10) verjüngt, sodass die im Wesentlichen sphärische Sitzfläche (4) des Schraubenkopfes (3) beim Eindrehen der Knochenschraube (2) in den Knochen, unter Verschiebung der Knochenplatte (6), entlang einer Führungsfläche (17) des Führungsabschnitts (15) gleitet und in den Verblockungsabschnitt (12) geführt wird.

2. Fixationssystem (1) gemäß Anspruch 1, **dadurch gekennzeichnet, dass** der Verblockungsabschnitt (12) im Wesentlichen zylindrisch oder konisch ist.

3. Fixationssystem (1) gemäß Anspruch 1 oder 2, **dadurch gekennzeichnet, dass** sich der umformbare Vorsprung (14) im Querschnitt gesehen über den gesamten Umfang des Verblockungsabschnitts (12) erstreckt.

4. Fixationssystem (1) gemäß einem der Ansprüche 1 bis 3, **dadurch gekennzeichnet, dass** der umformbare Vorsprung (14) Teil eines umformbaren Gewindes ist.

5. Fixationssystem (1) gemäß einem der Ansprüche 1 bis 4, **dadurch gekennzeichnet, dass** der Führungsabschnitt (15) eine zur Lochachse (13) parallele Eingangsfläche (16) und eine schräge, zum Verblockungsabschnitt (12) führende Führungsfläche (17) aufweist.

6. Fixationssystem (1) gemäß einem der Ansprüche 1 bis 5, **dadurch gekennzeichnet, dass** der Schraubenkopf (3) im verblockten Zustand vollständig in dem Verblockungsabschnitt (12) aufgenommen ist.

7. Fixationssystem (1) gemäß einem der Ansprüche 1 bis 6, **dadurch gekennzeichnet, dass** als Schraubeneintrittsöffnung (8) ein Langloch vorgesehen ist.

8. Fixationssystem (1) gemäß Anspruch 7, **dadurch gekennzeichnet, dass** die Breite (b) des Langloches im Wesentlichen dem Durchmesser der Schraubenaustrittsöffnung (10) entspricht.

9. Fixationssystem (1) gemäß einem der Ansprüche 1 bis 8, **dadurch gekennzeichnet, dass** das Durchgangsloch eine sich zwischen der Schraubeneintrittsöffnung (8) und der Schraubenaustrittsöffnung (10) erstreckende zylindrisch geformte Seitenwand (18) aufweist.

## Claims

1. Fixation system (1) for a bone comprising:
- a bone screw (2) having a screw head (3) with an essentially spherical seat (4) on which a head thread (5) is built and
- a bone plate (6) for fixed-angle polyaxial blocking of the bone screw (2) comprising:
a topside of the plate (7) with a screw inlet opening (8) for inserting the bone screw (2),
an underside of the plate(9) provided for bone contact with a screw outlet opening (10),
a through hole (11) extending between the screw inlet opening (8) and the screw outlet opening (10),
a blocking section (12) comprising at least one formable protrusion (14), formable by screwing in the bone screw (2) for stable blocking of the bone screw (2) at different angles to the hole axis (13),
**characterized in that**
the through hole (11) comprises a guide section (15) which tapers from the screw inlet opening (8) in the direction of the screw outlet opening (10), so that the essentially spherical seat (4) of the screw head (3) slides along a guide surface (17) of the guide section (15) when the bone screw (2) is screwing into the bone, displacing the bone plate (6), and is guided into the blocking section (12).

2. Fixation system (1) according to claim 1, **characterized in that** the blocking section (12) is essentially cylindrical or conical.

3. Fixation system (1) according to claim 1 or 2, **characterized in that** the formable protrusion (14), from a cross-sectional view, extends over the entire circumference of the blocking section (12).

4. Fixation system (1) according to one of claims 1 to 3, **characterized in that** the formable protrusion (14) is part of a formable thread.

5. Fixation system (1) according to one of claims 1 to 4, **characterized in that** the guide section (15) comprises an inlet surface (16) parallel to the hole axis (13) and an inclined guide surface (17) leading to the blocking section (12) .

6. Fixation system (1) according to one of claims 1 to 5, **characterized in that** the screw head (3) is completely received in the blocking section (12) in the blocked state.

7. Fixation system (1) according to one of claims 1 to 6, **characterized in that** a slot is provided as a screw inlet opening (8).

8. Fixation system (1) according to claim 7, **characterized in that** the width (b) of the slot essentially corresponds to the diameter of the screw outlet opening (10).

9. Fixation system (1) according to one of claims 1 to 8, **characterized in that** the through hole comprises a cylindrically shaped side wall (18) extending between the screw inlet opening (8) and the screw outlet opening (10).

## Revendications

1. Système de fixation (1) pour un os comprenant :
- une vis à os (2) avec une tête de vis (3) avec une surface d'assise (4) sensiblement sphérique, sur laquelle est réalisé un filet de tête (5) et
- une plaque orthopédique (6) pour le blocage polyaxial à angle stable de la vis à os (2), présentant :
une face supérieure de plaque (7) avec une ouverture d'entrée de vis (8) pour introduire la vis à os (2),
une face inférieure de plaque (9) prévue pour le contact avec l'os avec une ouverture de sortie de vis (10),
un trou débouchant (11) s'étendant entre l'ouverture d'entrée de vis (8) et l'ouverture de sortie de vis (10),
une partie de blocage (12), qui présente au moins une saillie déformable (14) par vissage de la vis à os (2) pour le blocage stable de la vis à os (2) dans différents angles par rapport à l'axe de trou (13),
**caractérisé en ce que**
le trou débouchant (11) présente une partie de guidage (15), qui s'amincit à partir de l'ouverture d'entrée de vis (8) en direction de l'ouverture de sortie de vis (10), de sorte que la surface d'assise (4) sensiblement sphérique de la tête de vis (3) lors du vissage de la vis à os (2) dans l'os, avec déplacement de la plaque orthopédique (6), glisse le long d'une surface de guidage (17) de la partie de guidage (15) et est guidée dans la partie de blocage (12).

2. Système de fixation (1) selon la revendication 1, **caractérisé en ce que** la partie de blocage (12) est sensiblement cylindrique ou conique.

3. Système de fixation (1) selon la revendication 1 ou 2, **caractérisé en ce que** la saillie déformable (14) vue en coupe transversale s'étend sur toute la périphérie de la partie de blocage (12).

4. Système de fixation (1) selon l'une quelconque des revendications 1 à 3, **caractérisé en ce que** la saillie déformable (14) fait partie d'un filet déformable.

5. Système de fixation (1) selon l'une quelconque des revendications 1 à 4, **caractérisé en ce que** la partie de guidage (15) présente une surface d'entrée (16) parallèle à l'axe de trou (13) et une surface de guidage (17) inclinée, menant à la partie de blocage (12).

6. Système de fixation (1) selon l'une quelconque des revendications 1 à 5, **caractérisé en ce que** la tête de vis (3) dans l'état bloqué est reçue entièrement dans la partie de blocage (12).

7. Système de fixation (1) selon l'une quelconque des revendications 1 à 6, **caractérisé en ce qu'**un trou oblong est prévu comme ouverture d'entrée de vis (8).

8. Système de fixation (1) selon la revendication 7, **caractérisé en ce que** la largeur (b) du trou oblong correspond sensiblement au diamètre de l'ouverture de sortie de vis (10).

9. Système de fixation (1) selon l'une quelconque des revendications 1 à 8, **caractérisé en ce que** le trou débouchant présente une paroi latérale (18) formée de manière cylindrique s'étendant entre l'ouverture d'entrée de vis (8) et l'ouverture de sortie de vis (10).
